# EUROPÄISCHE PATENTSCHRIFT

(11) **EP 1 181 894 B1**
(45) Veröffentlichungstag und Bekanntmachung des Hinweises auf die Patenterteilung: **27.04.2005**
(21) Anmeldenummer: 01117804.3
(22) Anmeldetag: 21.07.2001
(51) Int. Cl.: A61B 17/68

(54) **Osteosynthesemittel**
Osteosynthesis means
Moyens d'ostéosynthèse

(30) Priorität: 24.08.2000 DE 20014648 U
(43) Veröffentlichungstag der Anmeldung: 27.02.2002
(73) Patentinhaber: Stryker Trauma GmbH, 24232 Schönkirchen (DE)
(72) Erfinder: Seemann, Michael, 24161 Altenholz (DE)
(74) Vertreter: Patentanwälte Hauck, Graalfs, Wehnert, Döring, Siemons, Schildberg

(56) Entgegenhaltungen:
- EP-A- 0 298 400
- EP-A- 0 827 717
- EP-A- 0 885 598
- US-A- 2 489 870
- PATENT ABSTRACTS OF JAPAN vol. 1998, no. 05, 30. April 1998 (1998-04-30) & JP 10 014936 A (HOMUZU GIKEN:KK), 20. Januar 1998 (1998-01-20)

## Beschreibung

Die Erfindung bezieht sich auf ein Osteosynthesemittel, z.B. eine Kondylenschraube nach dem Oberbegriff des Anspruchs 1.

Ein suprakondylarer oder retrograder Knochennagel ist aus EP-A-0 827 717 bekannt geworden. Er weist einen länglichen Schaft auf, der im Verhältnis zu sonstigen Femurnägeln relativ kurz ausgeführt ist und der über eine Bohrung, die distal zwischen den Kondylen mündet und im Übrigen dem Knochenkanal folgt, eingetrieben wird. Damit eine Fraktur im Kondylenbereich gut versorgt werden kann, ist eine sogenannte Kondylenschraube vorgesehen, die durch eine Querbohrung des Knochennagels hindurchführbar ist. Die Kondylenschraube weist einen Mutterabschnitt und einen Schaftabschnitt auf, die miteinander verschraubt werden. Jeweils am Ende haben die Abschnitte eine flanschartige Erweiterung, die sich gegen die zugekehrte Knochenseite anlegt. Mit Hilfe einer derartigen Schraube lassen sich die Knochenfragmente wirksam komprimieren und am Nagel halten. Letzterer ist normalerweise als sogenannter Verriegelungsnagel ausgeführt.

Es hängt von der Lage der Enden der Kondylenschraube und der Außenkontur des Knochens ab, in welchem Ausmaß die flanschartige Erweiterung am Knochen anliegt. Es kann sehr leicht geschehen, dass nur kleine Flächenbereiche zur Anlage kommen und dadurch einen unerwünscht hohen Pressdruck erzeugen.

Aus JP-A-1 001 49 36 ist ein Osteosynthesemittel bekannt geworden, das aus einem Mutterteil und einem Schaftteil zusammengesetzt ist, die beide einen Flanschabschnitt aufweisen. Ein Flanschabschnitt ist als separates plattenförmiges Lagerteil ausgebildet, das einen Lagersitz bildet für einen balligen Endabschnitt des Mutterteils. Aus EP-A-0298400 ist bekannt geworden, auf einen Kirschnerdraht eine ringförmige Scheibe aufzustecken, die auf einer Seite eine ballige Lagerfläche aufweist zur Aufnahme einer Kugel, welche ebenfalls auf den Kirschnerdraht aufgeschoben wird.

Der Erfindung liegt die Aufgabe zugrunde, ein Osteosynthesemittel zu schaffen, das eine optimale Anpassung an den Knochen ermöglicht und das so ausgeführt ist, daß kein unfreiwilligen Lösen während der Anbringung der Schraube oder danach stattfindet.

Diese Aufgabe wird durch die Merkmale des Patentanspruchs 1 gelöst.

Bei der erfindungsgemäßen Schraube ist mindestens ein Flanschabschnitt als separates, ringförmiges Lagerteil vorgesehen, das auf einem balligen Endabschnitt des Mutterteils oder des Schaftteils beweglich gelagert ist. Die schwenkbare Lagerung des Flanschabschnitts ermöglicht eine Beweglichkeit zu allen Richtungen hin, so dass eine optimale Anpassung an die Gegebenheiten erfolgt und damit eine minimale Flächenpressung am Knochen verursacht wird.

Das Lagerteil ist so geformt, dass es schnappend auf den balligen Endabschnitt aufsetzbar ist. Es muss jedoch dafür gesorgt werden, dass die Schnappverbindung so ausgeführt ist, dass kein unfreiwilliges Lösen während der Anbringung der Schraube oder danach stattfindet. Insbesondere darf in Zugrichtung kein Herausschnappen der beiden Teile stattfinden.

Eine besonders einfache konstruktive Ausführung besteht erfindungsgemäß darin, dass das Lagerteil eine Lagerpfanne ist, die einen Schlitz aufweist, der eine federnde Aufweitung der Lagerpfanne zulässt. Hierbei besteht jedoch unter Umständen die Gefahr, dass eine Aufweitung auch durch Zugbelastung stattfindet. Daher sieht eine weitere Ausgestaltung der Erfindung vor, dass der Schlitz so geformt ist, dass die dem Schlitz zugekehrten Enden der Lagerpfanne nur ein begrenztes Maß voneinander fort bewegt werden können. Ein derartiger Schlitz kann zum Beispiel S- oder Z-förmig sein. Sobald das Auseinanderbewegen der Enden der ringförmigen Lagerpfanne ein vorgegebenes Maß überschreitet, kommen Abschnitte des Schlitzes zur Anlage und verhindern eine weitere Aufweitung.

Eine alternative Möglichkeit besteht auch darin, das Lagerteil nicht zu schlitzen, sondern achsparallele Vorsprünge (Stege) vorzusehen, die im Umfangsabstand am ringförmigen Lagerteil angeformt sind und einen Teil einer lagerpfannenartigen Ausnehmung bilden. Die erhöhter Vorsprünge (Stege) umgreifen den balligen Endabschnitt und federn beim Einsetzen des Endabschnitts leicht auf. Nach dem Montieren verhindern die Vorsprünge (Stege) ein unfreiwilliges Lösen des Endabschnitts aus dem Lagerteil.

Das erfindungsgemäße Osteosynthesemittel kann z.B. bei einem suprakondylaren Nagel verwendet werden, aber auch bei jedem anderen Verriegelungsnagel. Es ist aber auch möglich, das Osteosynthesemittel ohne einen Knochennagel zu verwenden, wenn eine entsprechende Versorgungssituation vorliegt.

Die Erfindung wird nachfolgend anhand eines in Zeichnungen dargestellten Ausführungsbeispiels näher erläutert.
- Fig. 1: zeigt die Seitenansicht eines Schaftteils einer Kondylenschraube nach der Erfindung.
- Fig. 2: zeigt einen Längsschnitt durch die Darstellung nach Fig. 1.
- Fig. 3: zeigt die Seitenansicht eines Mutterteils einer Kondylenschraube nach der Erfindung.
- Fig. 4: zeigt einen Schnitt durch das Mutterteil nach Fig. 3.
- Fig. 5: zeigt eine Draufsicht auf das Lagerteil für die Kondylenschraube nach der Erfindung.
- Fig. 6: zeigt einen Schnitt durch die Darstellung nach Fig. 5 entlang der Linie 6-6.
- Fig. 7: zeigt einen Schnitt durch einen distalen Femurteil mit einem suprakondylaren Nagel und einer Kondylenschraube nach dem Stand der Technik.
- Fig. 8: zeigt die Draufsicht auf ein Lagerteil einer anderen Ausführungsform der Erfindung.
- Fig. 9: zeigt die Seitenansicht des Lagerteils nach Fig. 8.
- Fig. 10: zeigt einen Schnitt durch das Lagerteil nach Fig. 8 entlang der Linie 10-10.

Es sei vorangestellt, dass die Zeichnungen nicht maßstäblich sind.

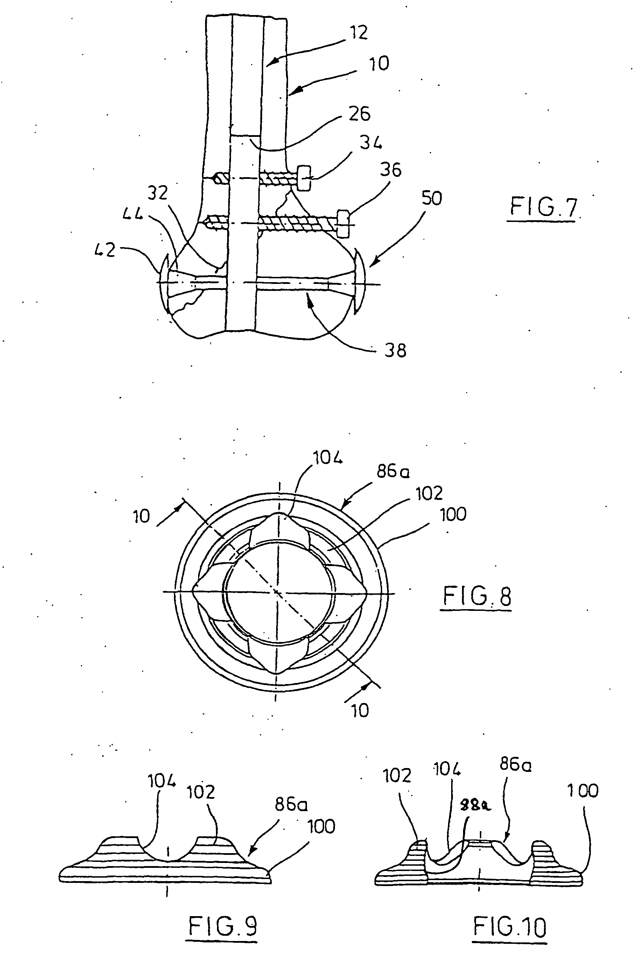

In Fig. 7 ist der distale Bereich eines Femurs 10 dargestellt, der einen Knochennagel 12 aufnimmt. Dieser ist durch eine Bohrung, die von subkondylär retrograd vorgenommen ist, eingeführt. Dies ist in DE 296 15 482 ausführlich erläutert. Es wird ausdrücklich auf die Gebrauchsmusterschrift Bezug genommen.

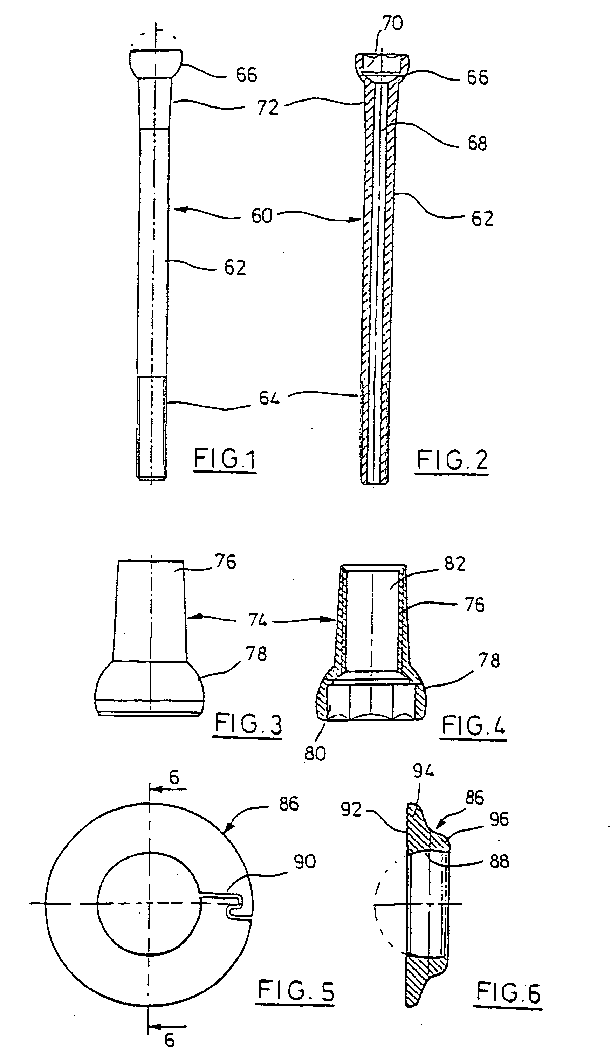

Der distale Bereich des Knochennagels 12 ist mit drei Querbohrungen versehen. In Fig. 7 (Stand der Technik) ist ferner zu erkennen, dass der Kondylenbereich des Femurs durch eine schräg verlaufende Fraktur mit der Bruchlinie 32 beschädigt ist. Zwei Knochenschrauben 34, 36 sind durch zwei der drei transversalen Bohrungen hindurchgeführt und dienen zur Verankerung des Nagels 12 im Femur 10. Durch die distal gelegene Transversalbohrung ist eine Kondylenschraube 38 hindurchgeführt, die aus einem Schaftteil 50 und einem Mutterteil 42 zusammengesetzt ist. Beide Teile 42, 50 sind aufeinandergeschraubt (wobei das Gewinde nicht zu erkennen ist). Mutterteil 42 und Schaftteil 50 weisen flanschartige Köpfe auf, an die sich konische Abschnitte 44 anschließen. Mit Hilfe der Kondylenschraube 38 lässt sich eine Kompression durchführen. Die flanschartigen Köpfe sind starr mit den zugeordneten Teilen verbunden. Die Teile der Kondylenschraube nach den Figuren 1 bis 6 haben einen ähnlichen Aufbau, sind jedoch in besonderer Weise gestaltet. Dies wird im Nachfolgenden näher beschrieben.

Der Schaftteil einer Kondylenschraube ist mit 60 bezeichnet und in den Figuren 1 und 2 dargestellt. Er weist einen glatten zylindrischen Schaftabschnitt 62, einen endseitigen Gewindeabschnitt 64 und einen balligen oder kugelabschnittförmigen Kopf 66 auf. Durch den Schaftteil 60 erstreckt sich axial durchgehend eine Bohrung 68. Im Kopf 66 befindet sich ein Inriensechskant 70 für den Eingriff mit einem Drehwerkzeug. Nahe dem Kopf 66 weist der Schaftteil 60 einen konischen Abschnitt 72 auf.

Ein Mutterteil 74 ist in den Figuren 3 und 4 dargestellt. Es weist einen konischen Gewindeabschnitt 76 mit einem Innengewinde auf und einen balligen oder kugelabschniaförmigen Kopf 78 mit einem Innensechskant 80. Das Mutterteil 74 weist ebenfalls eine durchgehende axiale Bohrung 82 auf. Wie erkennbar, sind Schaftteil 60 und Mutterteil 74 mit unterschiedlichem Maßstab dargestellt, denn das Mutterteil 74 wird im Gebrauch auf den Gewindeabschnitt 64 aufgeschraubt, wobei das freie Ende des konischen Gewindeabschnitts 76 einen Durchmesser aufweist, der gleich dem Durchmesser des glatten Schaftabschnitts 62 ist.

Auf die balligen Köpfe 66 bzw. 78 wird ein Lagerteil 86 geschnappt, dessen Kontur aus den Figuren 5 und 6 zu erkennen ist. Das Lagerteil 86 in Form einer Lagerpfanne ist am Umfang kreisrund und weist einen inneren kugeligen oder balligen Lagerabschnitt 88 auf. Seite Abmessung ist derart, dass annähernd passend der ballige Kopf 66 oder der ballige Kopf 78 von Schaftteil 60 bzw. Mutterteil 74 aufgenommen werden kann. Dadurch kann das Lagerteil 86 auf dem zugeordneten Kopf allseitig schwenken. Man erkennt, dass gemäß Fig. 7 dadurch eine optimale Anlage des Lagerteils 86 am Kondylenabschnitt des Femurs 10 ermöglicht wird.

Wie aus Fig. 5 zu erkennen, weist das Lagerteil 86 einen durchgehenden Z-förmigen Schlitz 90 auf. Dieser Schlitz ermöglicht ein Aufschnappen des Lagerteils 86 auf den Kopf 66 bzw. 78, indem die zugekehrten Enden des ringförmigen Lagerteils 86 etwas auseinanderbewegt werden. Es versteht sich, dass das Lagerteil 86 aus entsprechendem federndem Material besteht. Man erkennt ferner, dass die Form des Schlitzes 90 verhindert, dass die Enden des Lagerteils 86 um mehr als das Maß der Breite des Schlitzes 90 auseinanderbewegt werden. Daher kann beim Komprimieren ein nicht unerheblicher Druck auf das Lagerteil 86 vom Mutterteil bzw. Schaftteil ausgeübt werden, ohne dass die Teile voneinander getrennt werden.

Aus Fig. 6 ist zu erkennen, dass die dem Knochen zugewandte Seite der Lagerscheibe 86 plan ist, wie bei 92 dargestellt. Die gegenüberliegende Seite weist eine gerundete Kontur auf, welche aus einem der Fläche 92 benachbarten Flanschabschnitt 94 besteht und einem Bundabschnitt 96 zur anderen Seite des Lagerteils 86 hin.

Statt einer Kondylenschraube können auch zwei oder drei verwendet werden, wenn die zu versorgende Fraktur dies erfordert.

Das Lagerteil 86a nach Fign. 8 bis 10 besitzt einen ringförmigen Abschnitt 100 vergleichbar dem ringförmigen Abschnitt 94 des Lagerteils 86 nach den Figuren 5 und 6. Aus dem ringförmigen Abschnitt 100 sind vier Vorsprünge 102 herausgeformt, die sich annähernd achsparallel vom ringförmigen Abschnitt 100 fort erstrecken und im annähernd 90°-Abstand angeordnet sind. Zwischen diesen sind gerundete Vertiefungen 104 geformt. In der Seitenansicht (Fig. 9) sind die Vertiefungen halbkreisförmig. Die Vorsprünge 102 bilden zusammen mit dem ringförmigen Abschnitt 100 eine Lagerpfanne 88a. Diese ist im Bereich der Vorsprünge 102 durch die Vertiefungen 104 unterbrochen. Über den Bereich der Vorsprünge 102 kann ein balliger Endabschnitt, wie der Kopf 66 nach den Figuren 1 bis 4 in die Lagerpfanne 88a eingesetzt werden. Dabei verformen sich die Vorsprünge 102 federnd ein wenig radial nach außen und schnappen anschließend über den Kopf, so dass das Lagerteil 86a unverlierbar auf dem Endabschnitt gesichert ist. Im Übrigen entspricht die Funktion des Lagerteils 86a vollständig derjenigen des Lagerteils 86.

Das in den Figuren 1 bis 6 und 8 bis 10 dargestellte Osteosynthesemittel ist für die verschiedensten Versorgungsfälle bei Knochen einsetzbar, auch ohne einen Knochennagel, etwa nach Fig. 7. Wird sie bei einem Fall gemäß Fig. 7 eingesetzt, wird die Schraube 38 durch eine erfindungsgemäße Schraube ersetzt. Auch die Schrauben 34, 36 nach Fig. 7 können durch eine erfindungsgemäße Schraube ersetzt werden. Die Köpfe dieser Schraube gemäß Fign. 5, 6 und 8 bis 10 ermöglichen mit dem Lagerteil durch seine Verschwenkbarkeit eine optimale Anlage der Lagerteile an den Knochen. Dies ist z. B. durch die Köpfe der Schrauben 34, 36 in Fig. 7 nicht gewährleistet.

## Patentansprüche

1. Osteosynthesemittel, insbesondere Kondylenschraube, das aus einem Mutterteil (74) und einem Schaftteil (60) zusammengesetzt ist, die beide einen Flanschabschnitt aufweisen, wobei mindestens ein Flanschabschnitt als separates Teil und als Lagerteil (86, 86a) ausgebildet ist, das einen Lagersitz bildet für einen balligen Endabschnitt (66, 78) des Mutterteils (74) oder des Schaftteils (60) und dieses beweglich lagert, **dadurch gekennzeichnet dass** das ringförmige Lagerteil (86, 86a) so geformt ist, dass es schnappend auf den balligen Endabschnitt (66, 78) aufsetzbar ist.

2. Osteosynthesemittel nach Anspruch 1, **dadurch gekennzeichnet, dass** das ringförmige Lagerteil (86) eine Lagerpfanne ist, die einen Schlitz (90) aufweist, der eine federnde Aufweitung der Lagerpfanne (86) zulässt.

3. Osteosynthesemittel nach Anspruch 2, **dadurch gekennzeichnet, dass** der Schlitz (90) so geformt ist, dass die dem Schlitz (90) zugekehrten Enden der Lagerpfanne nur ein begrenztes Maß voneinander fort bewegt werden können.

4. Osteosynthesemittel nach Anspruch 3, **dadurch gekennzeichnet, dass** der Schlitz (90) S- oder Z-förmig ist.

5. Osteosynthesemittel nach Anspruch 1 **dadurch gekennzeichnet, dass** der Lagersitz (88a) von in Umfangsrichtung beabstandeten, achsparallelen Vorsprüngen (102) begrenzt ist, die eine Öffnung für das Einsetzen des balligen Endabschnitts (66, 78) begrenzen und während des Einsetzvorgangs vorübergehend radial nach außen verformt werden.

6. Osteosynthesemittel nach Anspruch 5, **dadurch gekennzeichnet, dass** zwischen den Vorsprüngen (102) gerundete Vertiefungen (104) vorgesehen sind, die in einem ringförmigen Abschnitt des ringförmigen Lagerteils (86) geformt sind annähernd parallel zur Achse des Lagerteils (86a).

## Claims

1. An osteosynthetic means, particularly a condylus screw, which is composed of a female-type part (74) and a shank part (60) which which both have a flanged portion at least one flanged portion is a separate bearing member (86, 86a) which defines a bearing seat for a ball-shaped end portion (66, 78) of the female-type part (74) or the shank part (60), and which is movably supported, **characterized in that** the annular bearing member (86, 86a) is formed such that it can be snappingly plugged onto the ball-shaped end portion (66, 78).

2. The osteosynthetic means according to claim 1, **characterized in that** the bearing member (86) is a bearing cup (86) which is provided and which has a slot (90) permitting to resiliently enlarge the bearing cup (86).

3. The osteosynthetic means according to claim 2, **characterized in that** the slot (90) is shaped in such a way that the ends of the bearing cup (86) which face the slot (90) can be moved away from each other only to a limited degree.

4. The osteosynthetic means according to claim 3, **characterized in that** the slot (90) is of a S shape or Z shape.

5. The osteosynthetic means according to claim 1, **characterized in that** the bearing seat (88a) is bordered by circumferentially spaced-apart, axially parallel projections (102) which limit an opening for inserting the ball-shaped end portion (66, 78) and are temporarily radially deformed outwardly during the insertion operation.

6. The osteosynthetic means according to claim 5, **characterized in that** rounded depressions (104) are provided between the protrusions (102), which are formed in a ring-shaped portion of the ring-shaped bearing member (86) approximately in parallel with the axis of the bearing member (86a).

## Revendications

1. Moyens d'ostéosynthèse, en particulier vis condylienne, qui sont composés d'une partie femelle (74) et d'une partie mâle (60), qui présentent toutes deux une section en forme de bride, au moins une section en forme de bride étant formée en tant que pièce distincte et en tant que pièce de palier (86 , 86a), qui forme un siège de palier pour une section d'extrémité bombée (66, 78) de la partie femelle (74) ou de la partie mâle (60) et abrite celle-ci de manière mobile, **caractérisés en ce que** la pièce de palier annulaire (86, 86a) est formée de telle sorte qu'elle puisse être montée par encliquetage sur la section d'extrémité bombée (66, 78).

2. Moyens d'ostéosynthèse selon la revendication 1, **caractérisés en ce que** la pièce de palier annulaire (86) est un coussinet, qui présente une entaille (90), qui permet un élargissement élastique du coussinet (86).

3. Moyens d'ostéosynthèse selon la revendication 2, **caractérisés en ce que** l'entaille (90) est formée de telle sorte que les extrémités du coussinet tournées vers l'entaille (90) ne puissent bouger les unes par rapport aux autres que de façon limitée.

4. Moyens d'ostéosynthèse selon la revendication 3, **caractérisés en ce que** l'entaille (90) est en forme de S ou de Z.

5. Moyens d'ostéosynthèse selon la revendication 1, **caractérisés en ce que** le siège de palier (88a) est limité par des saillies (102) parallèles à l'axe et écartées dans la direction circonférentielle, saillies qui limitent une ouverture destinée à l'introduction de la section d'extrémité bombée (66, 78) et qui sont déformées, pendant le processus d'introduction, de manière temporaire radialement vers l'extérieur.

6. Moyens d'ostéosynthèse selon la revendication 5, **caractérisés en ce que** des creux arrondis (104) sont prévus entre les saillies (102), creux qui sont formés dans une section annulaire de la pièce de palier annulaire (86) approximativement parallèlement à l'axe de la pièce de palier (86a).
